(19) Europäisches Patentamt
European Patent Office
Office européen des brevets

(11) EP 0 347 678 B1

(12) EUROPÄISCHE PATENTSCHRIFT

(45) Veröffentlichungstag und Bekanntmachung des Hinweises auf die Patenterteilung:
**26.02.1997 Patentblatt 1997/09**

(21) Anmeldenummer: **89110528.0**

(22) Anmeldetag: **10.06.1989**

(51) Int Cl.⁶: **C07D 211/90**, C07D 491/04, C07C 229/00, C07C 311/00, C07C 333/00, C07C 335/00, C07C 271/00, A61K 31/44, C07C 275/00, C07D 211/98

(54) **1,4-Dihydropyridin-threonin-Derivate und Verfahren zu ihrer Herstellung**

1,4-Dihydropyridine threonin derivatives and process for their preparation

Dérives de 1,4-dihydropyridine-thréonine et procédé pour leur préparation

(84) Benannte Vertragsstaaten:
**AT BE CH DE ES FR GB GR IT LI LU NL SE**

(30) Priorität: **24.06.1988 DE 3821334**

(43) Veröffentlichungstag der Anmeldung:
**27.12.1989 Patentblatt 1989/52**

(60) Teilanmeldung: **94112407.5**

(73) Patentinhaber: **BAYER AG**
**51368 Leverkusen (DE)**

(72) Erfinder:
- **Stoltefuss, Jürgen, Dl.**
  **D-5657 Haan (DE)**
- **Bechem, Martin, Dr.**
  **D-5600 Wuppertal 1 (DE)**
- **Gross, Rainer, Prof.- Dr.**
  **D-5600 Wuppertal 1 (DE)**
- **Hebisch, Siegbert, Dr.**
  **D-5600 Wuppertal 1 (DE)**
- **Schramm, Matthias, Dr.**
  **D-5000 Köln (DE)**

(56) Entgegenhaltungen:
**EP-A- 0 026 317** **DE-A- 3 714 438**

- **ANGEW. CHEM., Int. Ed. Engl. 30, (1991), S. GOLDMANN et al., pages 1559-1578.**

Bemerkungen:
Die Akte enthält technische Angaben, die nach dem Eingang der Anmeldung eingereicht wurden und die nicht in dieser Patentschrift enthalten sind.

EP 0 347 678 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die vorliegende Erfindung betrifft neue Dihydropyridin-Derivate und Verfahren zu ihrer Herstellung.

Es ist bereits bekannt geworden, daß man Dihydropyridincarbonsäuren aus den entsprechenden Dihydropyridincarbonsäure-β-cyanoethylestern darstellen kann (vgl. DOS 39 29 545).

Gegenstand der Erfindung sind Dihydropyridin-Derivate der Formel (I)

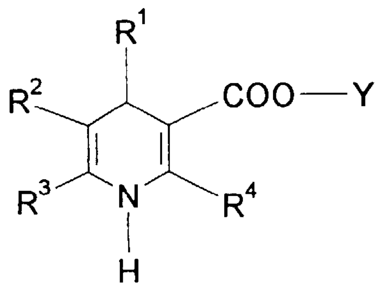

(I)

in welcher

$R^1$
- für geradkettiges oder verzweigtes Alkyl mit bis zu 4 Kohlenstoffatomen steht, oder
- für Cyclopropyl, Cyclopentyl oder Cyclohexyl steht, oder
- für Phenyl steht, das bis zu 2-fach gleich oder verschieden substituiert sein kann durch Nitro, Cyano, Trifluormethyl, Fluor, Chlor, Methyl, Methoxy, Trifluormethoxy, Difluormethoxy, gegebenenfalls durch Methyl, Fluor oder Chlor substituiertes Phenylsulfonyloxy, oder durch $C_1$-$C_4$-Alkoxy oder $C_1$-$C_4$-Alkylthio, die jeweils substituiert sein können durch Cyclohexyl oder Phenyl, das seinerseits durch Methyl, Fluor, Chlor oder Methoxy substituiert sein kann, oder
- für Pyridyl steht, und

$R^2$
- für Nitro, Cyano oder
- für eine Gruppe der Formel

COO-Z-$R^5$ oder

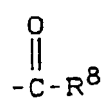

steht,

worin

$R^5$
- für Wasserstoff oder $C_1$-$C_6$-Alkyl steht, das gegebenenfalls durch Sauerstoff oder Schwefel unterbrochen sein kann und das gegebenenfalls durch Hydroxy, $C_1$-$C_4$-Alkoxycarbonyl oder Carboxy, oder durch Fluor, Chlor, Methyl oder Methoxy substituiertes Phenyl oder Phenoxy substituiert sein kann, oder
- für eine Gruppe der Formel

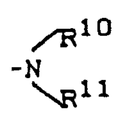

steht,
worin

$R^{10}$ und $R^{11}$ gleich oder verschieden sind und jeweils

- für Wasserstoff, für geradkettiges oder verzweigtes Alkyl mit bis zu 6 Kohlenstoffatomen stehen, das substituiert sein kann durch Fluor, Chlor, Hydroxy, Ethoxy, Methoxy, Carboxy, $C_1$-$C_4$-Alkoxycarbonyl oder durch Phenyl, welches seinerseits durch Trifluormethyl, Trifluormethoxy, $C_1$-$C_2$-Alkyl, Fluor, Chlor oder $C_1$-$C_2$-Alkoxy substituiert sein kann, oder
- für Cyclopropyl, Cyclopentyl oder Cyclohexyl stehen, oder
- für Phenyl stehen, das durch Fluor, Chlor, $C_1$-$C_2$-Alkyl, $C_1$-$C_2$-Alkoxy, Trifluormethyl, Trifluormethoxy, Difluormethoxy, Trifluormethylthio, Amino, $C_1$-$C_2$-Alkylamino substituiert sein kann, oder

$R^{10}$ und $R^{11}$ zusammen für Piperidinyl, Morpholinyl, Methyl-piperazinyl oder N-Benzylpiperazinyl stehen,

und

Z - für eine einfache Bindung oder eine geradkettige oder verzweigte Alkylenkette mit bis zu 6 C-Atomen steht und

$R^8$
- für geradkettiges oder verzweigtes Alkyl mit bis zu 4 Kohlenstoffatomen steht, oder
- für die Gruppe

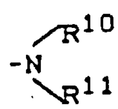

steht,
worin
$R^{10}$ und $R^{11}$ die oben angegebene Bedeutung haben,

und

$R^3$ - für geradkettiges, verzweigtes oder cyclisches Alkyl mit bis zu 4 Kohlenstoffatomen steht, oder

$R^2$ und $R^3$ gemeinsam einen Ring der Formel

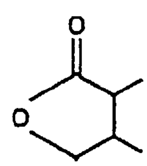

bilden,
und

$R^4$
- für geradkettiges oder verzweigtes Alkyl mit bis zu 4 Kohlenstoffatomen steht, oder
- für Cyclopropyl steht,

und

Y - für einen Rest

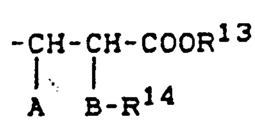

steht,
worin

A - für Wasserstoff oder Methyl steht,
und

B - für eine Gruppe der Formel -NH-, -NH-CO-, -NH-COO- oder -NH-$SO_2$- steht,
und

$R^{13}$- für geradkettiges oder verzweigtes Alkyl mit bis zu 6 Kohlenstoffatomen steht, das substituiert sein kann durch Hydroxy, Methoxy oder Phenyl,
und

$R^{14}$- Wasserstoff bedeutet, oder
- für geradkettiges oder verzweigtes Alkyl mit bis zu 6 Kohlenstoffatomen steht, das substituiert sein kann durch Hydroxy, $C_1$-$C_2$-Alkoxycarbonyl oder durch Phenyl, das seinerseits substituiert sein kann durch Methyl, Trifluormethyl, Fluor oder Chlor, oder
- für Cyclopropyl, Cyclopentyl oder Cyclohexyl steht, oder
- für Phenyl steht, das bis zu 2-fach gleich oder verschieden substituiert sein kann durch Nitro, Cyano, Fluor, Chlor, $C_1$-$C_2$-Alkyl, $C_1$-$C_4$-Alkoxy, $C_1$-$C_2$-Alkylthio, Carbamoyl, Carboxy, $C_1$-$C_4$-Alkoxycarbonyl, Trifluormethyl, Trifluormethoxy, $C_1$-$C_2$-Alkylsulfamoyl oder $C_1$-$C_2$-Alkylamino, oder
- für Pyridyl steht,

sowie deren Salze.

Die erfindungsgemäßen Verbindungen sind neu und besitzen wertvolle pharmakologische Eigenschaften. Sie beeinflussen die Kontraktionskraft des Herzens und können deshalb zur Bekämpfung von cardiovaskulären Erkrankungen verwendet werden. Durch die Möglichkeit, reine Enantiomere herzustellen, lassen sich die gewünschten pharmakologischen Eigenschaften der Verbindungen in unterschiedlichen Richtungen optimieren.

Die erfindungsgemäßen Verbindungen der allgemeinen Formel (I)

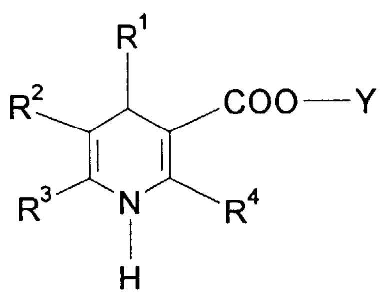

(I)

in welcher
$R^1$, $R^2$, $R^3$, $R^4$ und Y die oben angegebene Bedeutung haben, erhält man, indem man

[A] Aldehyde der allgemeinen Formel III

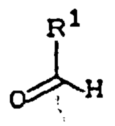

(III)

in welcher
$R^1$ die oben angegebene Bedeutung hat,
und Verbindungen der Formel (II)

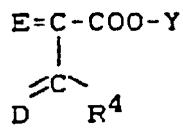

(II)

in welcher

$R^4$ und Y die oben angegebene Bedeutung haben,

D - für Sauerstoff steht, und

E in diesem Fall für zwei Wasserstoffatome steht,

gegebenenfalls nach Isolierung der entstehenden Benzylidenderivate mit Aminocrotonsäurederivaten der allgemeinen Formel (IV)

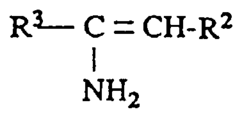

(IV)

in welcher
$R^2$ und $R^3$ die oben angegebene Bedeutung haben,
umsetzt,
oder indem man
[B] Aldehyde der allgemeinen Formel (III)

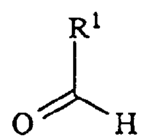

(III)

in welcher
$R^1$ die oben angegebene Bedeutung hat,
mit Verbindungen der allgemeinen Formel (V)

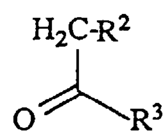

(V)

in welcher
$R^2$ und $R^3$ die oben angegebene Bedeutung haben,
entweder direkt oder nach Isolierung der entstehenden Benzylidenderivate der allgemeinen Formel (VI)

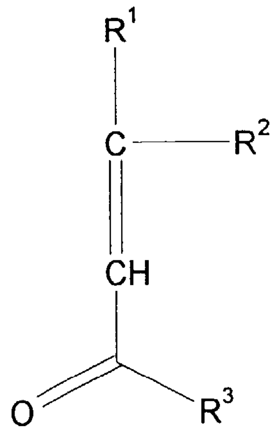

(VI)

in welcher

$R^1$, $R^2$ und $R^3$ die oben angegebene Bedeutung haben,

mit Verbindungen der allgemeinen tautomeren Formeln (II)

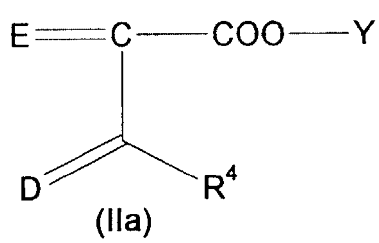

(IIa)

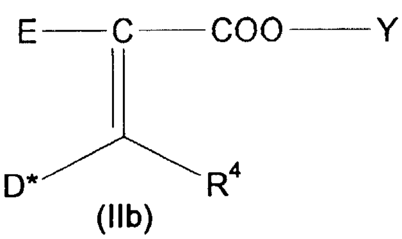

(IIb)

in welcher

$R^4$ und Ydie oben angegebene Bedeutung haben,

D für NH steht,

D* für NH steht, und

E für Wasserstoff steht, umsetzt.

Je nach Art der verwendeten Ausgangsstoffe können die Synthesevarianten für die erfindungsgemäßen Verbindungen durch folgende Formelschemata wiedergegeben werden.

[A]

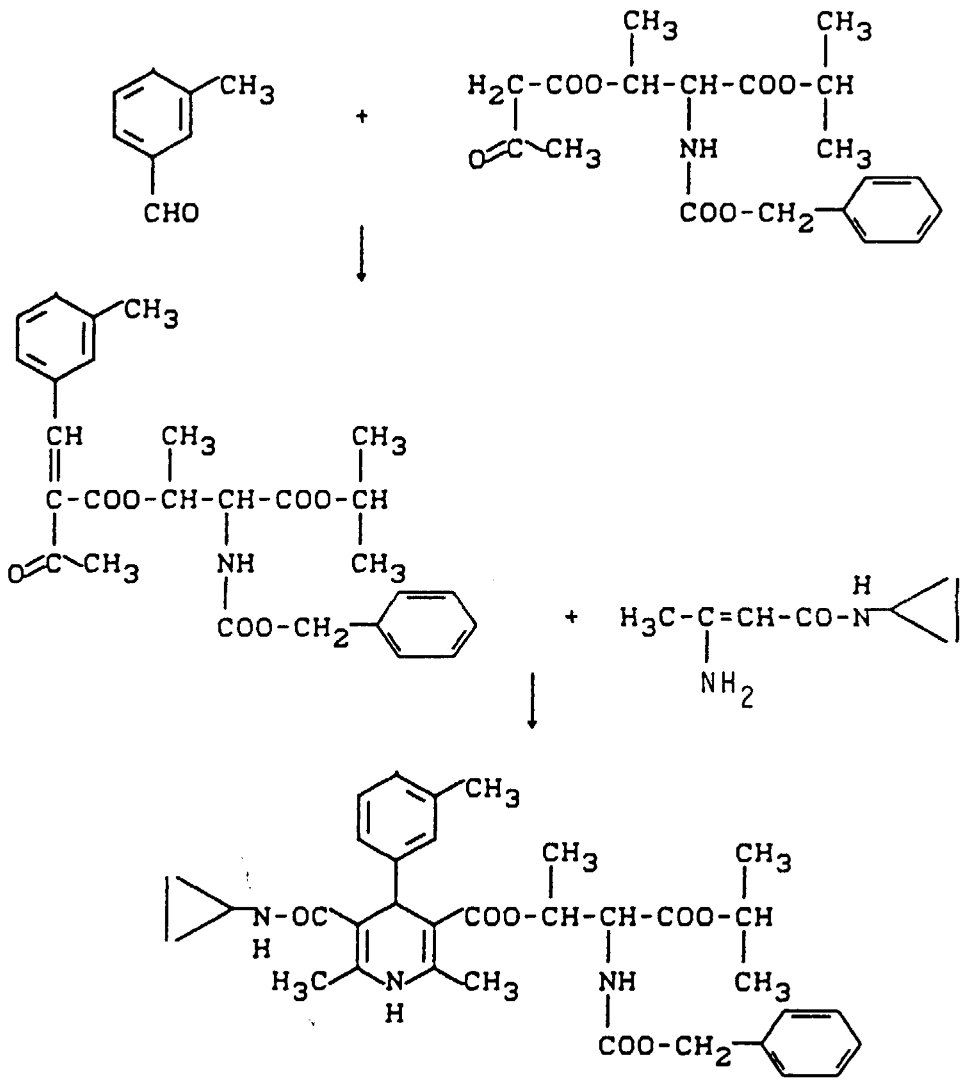

[B]

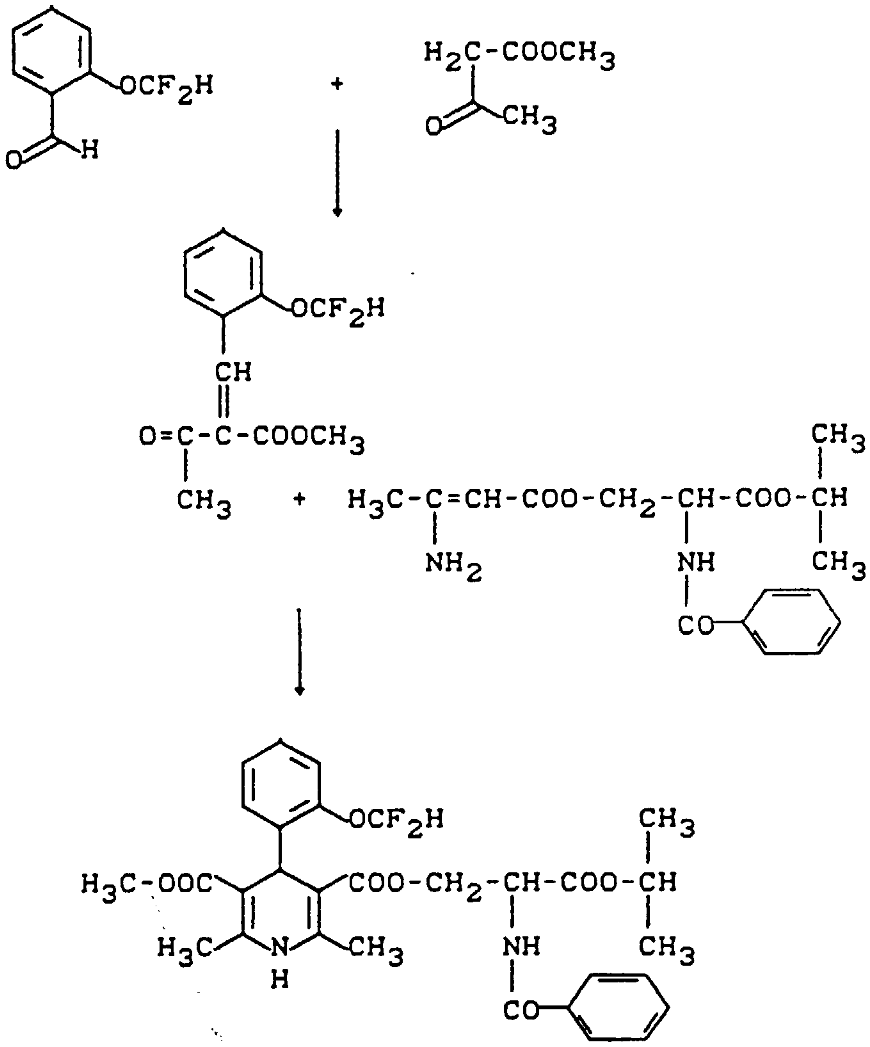

Als Lösemittel bei den Verfahren *[A]* und *[B]* kommen Wasser und alle inerten organischen Lösemittel in Frage, die sich unter den Reaktionsbedingungen nicht verändern. Hierzu gehören vorzugsweise Alkohole wie Methanol, Ethanol, Propanol, Isopropanol, Ether wie Diethylether, Dioxan, Tetrahydrofuran, Glykolmonomethylether oder Glykoldimethylether, oder Amide wie Dimethylformamid, Dimethylacetamid oder Hexamethylphosphorsäuretriamid, oder Eisessig, Dimethylsulfoxid, Acetonitril oder Pyridin, sowie Kohlenwasserstoffe wie Toluol, Xylol oder Benzol.

Die Reaktionstemperaturen können in einem größeren Bereich variiert werden. Im allgemeinen arbeitet man zwischen +10°C und +150°C, vorzugsweise zwischen +20°C und +100°C, insbesondere bei der Siedetemperatur des jeweiligen Lösemittels.

Die Umsetzung kann bei Normaldruck, aber auch bei erhöhtem oder erniedrigtem Druck durchgeführt werden. Im allgemeinen arbeitet man bei Normaldruck.

Bei der Durchführung der erfindungsgemäßen Verfahrensvarianten A und B ist das Verhältnis der an der Reaktion beteiligten Stoffe beliebig. Im allgemeinen arbeitet man jedoch mit molaren Mengen der Reaktanden. Die Isolierung und Reinigung der erfindungsgemäßen Substanzen erfolgt vorzugsweise derart, daß man das Lösemittel im Vakuum abdestilliert und den gegebenenfalls erst nach Eiskühlung kristallin erhaltenen Rückstand aus einem geeigneten Lösemittel umkristallisiert. In einigen Fällen kann es erforderlich sein, die erfindungsgemäßen Verbindungen durch Chromatographie zu reinigen.

Die Verbindungen der allgemeinen Formel (II) sind neu. Nur L-3-Oxobutansäure-(2-benzyloxycarbonyl-2-tert.butoxy-carbonyl-1-methyl)-ethylester wird als Schutzgruppe beim Aufbau von Aminosäuren eingesetzt [vgl. Bull. Chem. Soc. Jpn, 52 (16), 3111 - 3112].

Die neuen Verbindungen der Formel (II) können nach verschiedenen Verfahren hergestellt werden. So lassen sich

z.B. Verbindungen der Formel II in der

$R^4$ - für Methyl,

D - für Sauerstoff

E - für Wasserstoff steht, und

Y die oben angegebene Bedeutung hat,

herstellen, indem man

[A] Verbindungen der allgemeinen Formel (VIII)

HO-Y (VIII)

in welcher
Y die oben angegebene Bedeutung hat,
mit Diketen der allgemeinen Formel (IX)

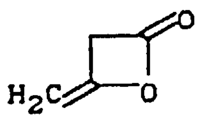

(IX)

umsetzt.

Die Verbindungen der Formel (II), in der

$R^4$ und Y die oben angegebene Bedeutung haben,

D - für Sauerstoff, und

E - für zwei Wasserstoffatome steht,

können auch hergestellt werden, indem man

[B] Verbindungen der Formel (VIII) mit Verbindungen der allgemeinen Formel (X)

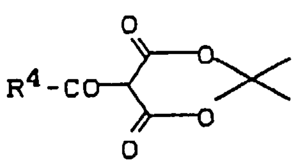

(X)

in welcher $R^4$ die oben angegebene Bedeutung hat, umsetzt.

Die als Ausgangsstoffe eingesetzten Aldehyde der allgemeinen Formel (III) sind bekannt oder können nach bekannten Methoden hergestellt werden [DOS 21 65 260; 24 01 665; T.D. Harris, G.P. Roth, J. Org. Chem. 44, 2004 (1979); W.J. Dale, H.E. Hennis, J. Am. Chem. Soc. 78, 2543 (1956); Chem. Abstr. 59, 13929 (1963)].

Die als Ausgangsstoffe eingesetzten Enamine der allgemeinen Formel (IV) sind bekannt oder können nach bekannten Methoden hergestellt werden [DOS 2 228 377; F.A. Glickman, A.C. Cope, J. Am. Chem. Soc. 67, 1017 (1945)].

Die als Ausgangsstoffe eingesetzten Yliden-β-ketocarbonsäurederivate der allgemeinen Formel (VI) sind bekannt oder können nach bekannten Methoden hergestellt werden [G. Jones "The Knoevenagel Condensation" in Organic Reactions Bd. XV, 204 (1967)].

Die Ausgangsstoffe der Formel (VIII) sind teilweise bekannt oder können nach bekannten Methoden hergestellt werden [vgl. Houben-Weyl, Methoden der organischen Chemie, Band 15, Teil1, E. Wünsch, Seite 46 ff.].

Die Verbindung der Formel (IX) und ihre Reaktionen sind bekannt (vgl. R. J. Clemens, Chem. Rev. 88, 241 (1986)).

Die Verbindungen der formel (X) sind zum Teil bekannt oder können nach üblicher Methode hergestellt werden (vgl. J. Org. Chem. 43, 1087 (1978)).

Die erfindungsgemäßen Verbindungen existieren in stereoisomeren Formen, die sich entweder wie Bild und Spiegelbild (Enantiomere) oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E. L. Eliel, Stereochemistry of Carbon Compounds, McGraw Hill, 1962).

Physiologisch unbedenkliche Salze können Salze der erfindungsgemäßen Verbindungen mit anorganischen oder organischen Säuren oder Basen sein. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure und Salze mit Basen wie beispielsweise Natriumhydroxid, Kaliumhydroxid, Bariumhydroxid, Ammoniumsalze, oder Salze mit organischen Basen wie Pyridin oder Triethylamin.

Die Verbindungen der allgemeinen Formel (I) können sowohl als Diastereomerengemisch als auch als reine Diastereomere vorliegen. Setzt man als Ausgangsmaterialien der Formel (VIII) reine L- oder D-Verbindungen ein, so erhält man aus diesen im Endeffekt Diastereomerenpaare der Formel (I), die sich überraschenderweise leicht nach bekannten Methoden wie Säulenchromatographie, fraktionierte Kristallisation oder Craigverteilung in die reinen Diastereomeren trennen lassen. [Zur Craigverteilung siehe beispielsweise "Verteilungsverfahren im Laboratorium, E. Hecker, Verlag Chemie GmbH, Weinheim, Bergstraße (1955)].

Gegenstand dieser Erfindung ist außerdem noch die Verwendung von Verbindungen der Formel I zur Herstellung von Dihydropyridincarbonsäuren der allgemeinen Formel (XI)

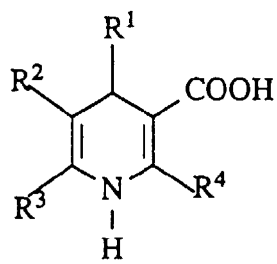

(XI)

in welcher

$R^1$, $R^2$, $R^3$ und $R^4$ die oben angegebene Bedeutung haben,

denn überraschenderweise wurde gefunden, daß sich der Rest Y der Verbindungen der Formel (I) im Gegensatz zu den sonst bekannten Dihydropyridinestern außerordentlich leicht abspalten läßt.

Setzt man reine Diastereomere der Formel (I) ein, so erhält man enantiomerenreine Carbonsäuren der Formel (XI), die ihrerseits nach bekannten Methoden in Ester, Amide, Nitrile, Lactone u.s.w. umgewandelt werden können und damit in einfacher Weise reine Enantiomere von Dihydropyridinen zugänglich werden.

Herstellungsbeispiele

Beispiel 1 (mit Diastereomerentrennung)

1,4-Dihydro-2,6-dimethyl-3-nitro-4-phenyl-pyridin-5-carbonsäure-(1R, 2S)-[1-methyl-2-methoxycarbonyl-2-(4-tolylsulfamoyl)]-ethylester

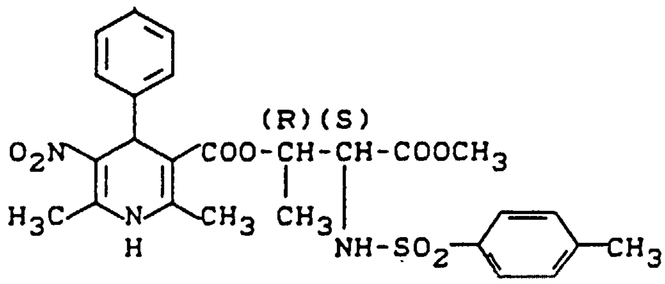

725 mg (3,8 mmol) Benzylidennitroaceton werden in 7 ml Ethanol mit 1,4 g (3,8 mmol) β-Aminocrotonsäure-(1R, 2S)-[1-methyl-2-methoxycarbonyl-2-(4-tolylsulfamoyl)]ethylester 3 Stunden gekocht. Es wird eingeengt und die beiden Diastereomeren werden durch Säulenchromatographie getrennt.
Man erhält 0,6 g eines gelbgefärbten Diastereomeren
$R_f$-Wert = 0,18 und 0,4 g eines Diastereomeren
$R_f$-Wert = 0,146
DC: Kieselgel Alurolle, Kieselgel 60, F 254,
Schichtdicke 0,2 mm (Merck)
Fließmittel: Methylenchlorid/Essigester im Volumenverhältnis 20:1

Beispiel 2 (mit Diastereomerentrennung)

1,4-Dihydro-2,6-dimethyl-4-(3-nitrophenyl)-pyridin-3carbonsäuremethylester-5-carbonsäure-(1R, 2S)-[1-methyl-2-methoxycarbonyl-2-(4-tolylsulfamoyl)]-ethylester

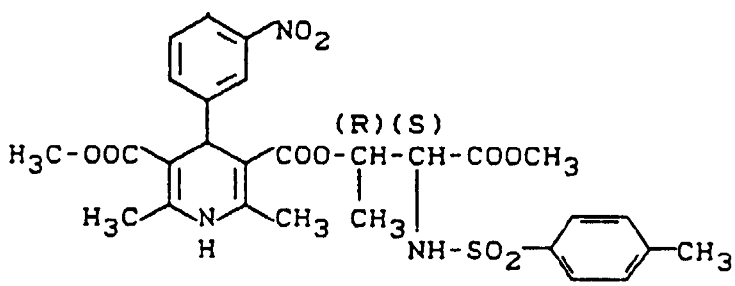

5 g (20 mmol) 2-(3-Nitrobenzyliden)-3-oxo-buttersäuremethylester werden in 40 ml Isopropanol mit 7,4 g (20 mmol) β-Aminocrotonsäure-(1R, 2S)-[1-methyl-2-methoxy-2-(4-tolylsulfamoyl)]-ethylester 5 Stunden gekocht. Es wird abgekühlt, die ausgefallenen Kristalle werden abgesaugt und mit Isopropanol gewaschen. Man erhält 4,8 g (39,9 % der Theorie) eines reinen Diastereomers vom Schmelzpunkt: 157 - 159°C (siehe Beispiel 4).
$[\alpha]^{20}_{589}$ = + 49,3° (C = 1,0:DMF)

Beispiel 3

1,4-Dihydro-2,6-dimethyl-3-nitro-4-phenyl-pyridin-5-carbonsäure-(1R, 2S)-(1-methyl-2-methoxycarbonyl-2-phenyl-carbamoyl)-ethylester

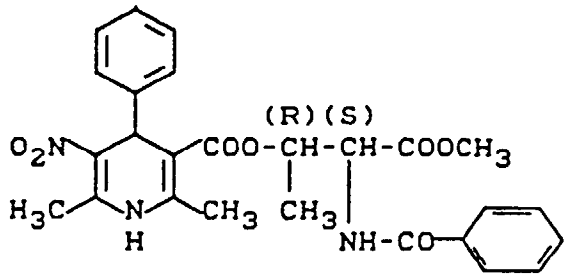

0,37 g (1,95 mmol) Benzyliden-nitroaceton werden mit 0,6 g (1,95 mmol) β-Aminocrotonsäure-(1R, 2S)-(1-methyl-2-methoxycarbonyl-2-phenyl-carbamoyl)-ethylester in 4 ml Ethanol 3 Stunden gekocht. Es wird eingeengt und das Diastereomerengemisch über eine Kieselgelsäule mit Toluol/Essigester gereinigt. Man erhält 0,7 g eines rotgelben Öls vom $R_f$-Wert 0,1 (Fließmittel: Methylenchlorid/Essigester 20:1).

Beispiel 4 (mit Diastereomerentrennung)

1,4-Dihydro-2,6-dimethyl-4-(3-nitrophenyl)-pyridin-3-carbonsäuremethylester-5-carbonsäure-(1R, 2S)-1-methyl-2-methoxy-carbonyl-2-(p-tolylsulfamoyl)-ethylester

Aus der Mutterlauge der Verbindung aus Beispiel 2 werden 3,6 g (29,9 % der Theorie) des zweiten Diastereomers durch Säulenchromatographie als Öl erhalten.
$R_f$-Wert: 0,38
Fließmittel: Toluol/Aceton (4:1)

Beispiel 5 (enantiomere Säure)

(-)1,4-Dihydro-2,6-dimethyl-4-(3-nitrophenyl)-pyridin-3,5-dicarbonsäure-3-methylester

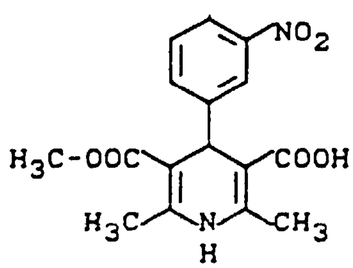

2 g (3,33 mmol) der Verbindung aus Beispiel 2 werden in 18 ml Methanol mit 1,22 ml (8,15 mmol) Diaza-bicycloundecan versetzt und 3 Stunden bei Raumtemperatur gerührt. Es wird eingeengt, der Eindampfrückstand wird mit Wasser versetzt und unter Rühren tropfenweise mit 10 %iger Salzsäure sauer gestellt. Das ausgefallene Festprodukt wird abgesaugt, mit Wasser und anschließend mit Ether gewaschen. Man erhält 1,02 g (88,5 % der Theorie) eines farblosen Produktes vom Schmelzpunkt 181 - 182°C unter Zersetzung.
$[\alpha]^{20}_{584}$ = -21°(c = 0,711, Aceton) (Enantiomerenüberschuß (ee) >99 %)

Analog den Beispielen 1 bis 4 wurden die in der folgenden Tabelle aufgeführten Beispiele erhalten.

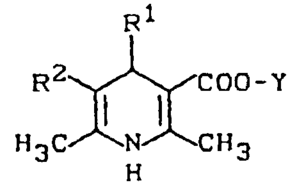

| Beisp.-Nr. | $R^1$ | $R^2$ | Y | Fp.: [°C] | $R_f$ (Laufmittel) |
|---|---|---|---|---|---|
| 6 | 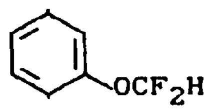 | $NO_2$ | 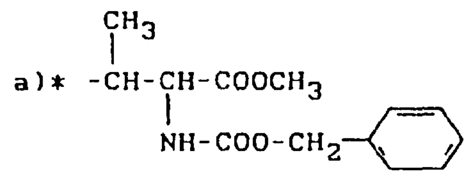 | | *1) 0,43/0,49 |
| 7 | 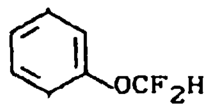 | $NO_2$ | 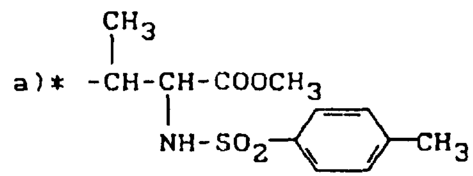 | Öl<br>175-178 | *1) 0,42 |
| 8 | 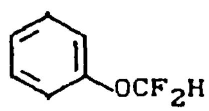 | $NO_2$ | 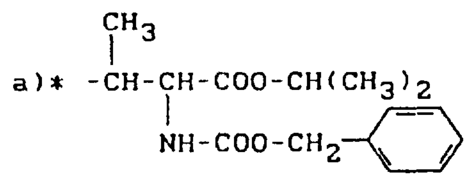 | 115-118 | *2) 0,56 |
| 9 | 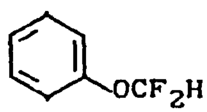 | $NO_2$ | 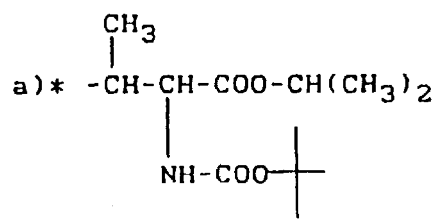 | Öl | *2) 0,46 |

(a = R, S; b = S, R)

| Beisp.-Nr. | $R^1$ | $R^2$ | Y | Fp.: [°C] | $R_f$ (Laufmittel) |
|---|---|---|---|---|---|
| 10 | 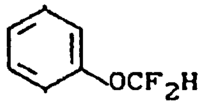 | $NO_2$ | a)* 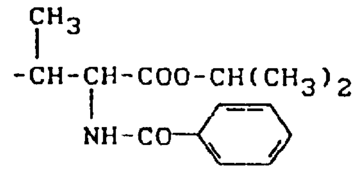 | Schaum | *2) 0.36/0.41 |
| 11 | 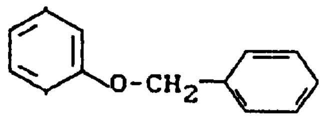 | $H_3C-OOC$ | a)* 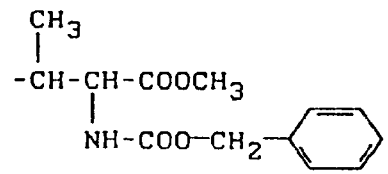 | Öl | *2) 0,2/0,24<br>*1) 0,54/0,59 |
| 12 | 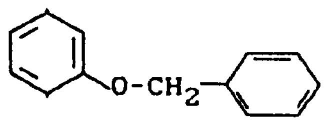 | $H_3C-OOC$ | a)* 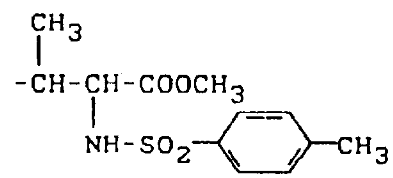 | 158-160 | *2) 0.38/0.41<br>*1) 0.53/0.59 |
| 13 | 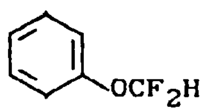 | $NO_2$ | a)* 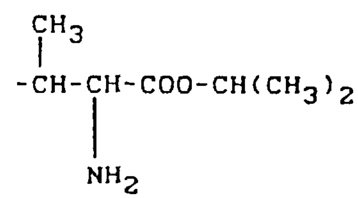 | Schaum | *2) 0,02/0,04<br>*3) 0,31 |

(a = R, S; b = S, R)

| Beisp.-Nr. | $R^1$ | $R^2$ | Y | Fp.: [°C] | $R_f$ (Laufmittel) |
|---|---|---|---|---|---|
| 14 | 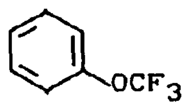 | $NO_2$ | a)* 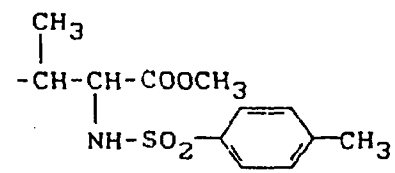 | 178 | *4) 0.16/0.21 |
| 15 | 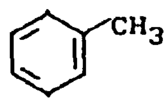 | $NO_2$ | a)* 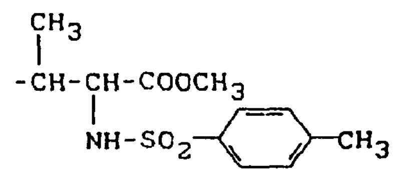 | Öl | *4) 0,16/0,19 |
| 16 | 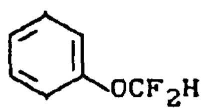 | $NO_2$ | a)* 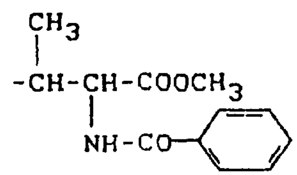 | Öl | *4) 0,09 |
| 17 | 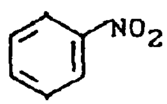 | $NO_2$ | a)* 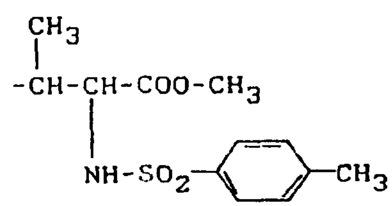 | Öl | *4) 0,29/0,36 |

(a = R, S; b = S, R)

| Beisp.-Nr. | $R^1$ | $R^2$ | Y | Fp.: [°C] | $R_f$ (Laufmittel) |
|---|---|---|---|---|---|
| 18 | 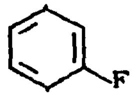 | $NO_2$ | a)* 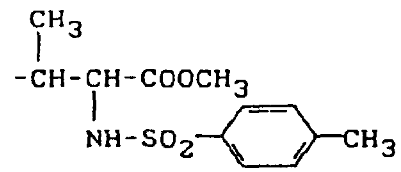 | 178-182 | *4) 0.22/0.92 *1) 0.22/0.29 |
| 19 | 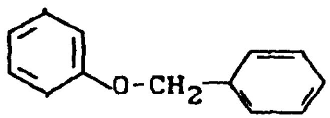 | $H_3C-OOC$ | a)* 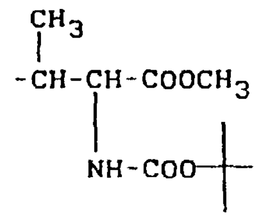 | Öl | *2) 0,24/0,29 *1) 0,56/0,62 |
| 20 | 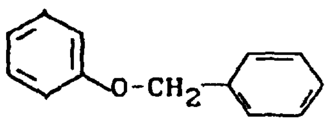 | $H_3C-OOC$ | a)* 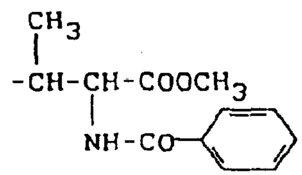 | Schaum | *2) 0,394/0,44 *1) 0,54/0,59 |
| 21 | 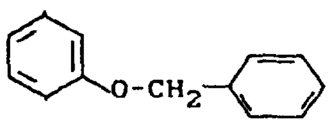 | $H_3C-OOC$ | a)* 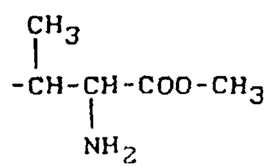 | Öl | *2) *1) 0,07/0,11 |

(a = R, S; b = S, R)

| Beisp.-Nr. | $R^1$ | $R^2$ | Y | Fp.: [°C] | $R_f$ (Laufmittel) |
|---|---|---|---|---|---|
| 22 | 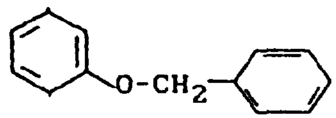 | $H_3C-OOC$ | a)* 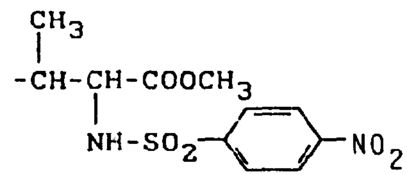 | Öl | *1) 0,63/0,66<br>*2) 0,4 |
| 23 | 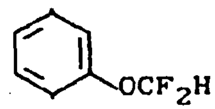 | $NO_2$ | a)* 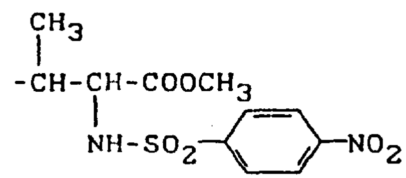 | 199-202 | *1) 0.55 |
| 24 | 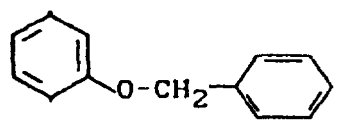 | $H_3C-OOC$ | a)* 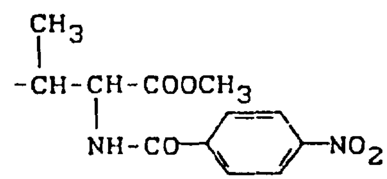 | Öl | *5) 0,53/0,59<br>*1) 0,89 |
| 25 | 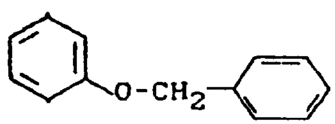 | $H_3C-OOC$ | a)* 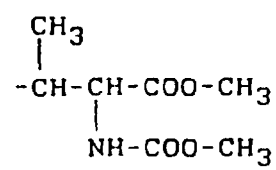 | Öl | *1) 0,44<br>*2) 0,18 |

(a = R, S; b = S,R)

| Beisp.-Nr. | $R^1$ | $R^2$ | Y | Fp.: [°C] | $R_f$ (Laufmittel) |
|---|---|---|---|---|---|
| 26 | 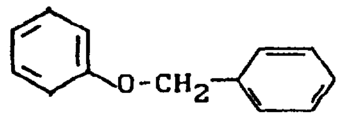 | $H_3C$-OOC | 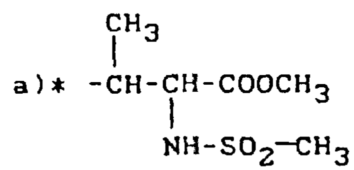 | Öl | *1) 0,43 *2) 0,18/0,23 |
| 27 | 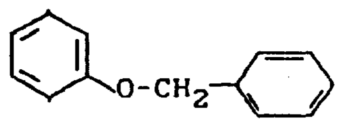 | $H_3C$-OOC | 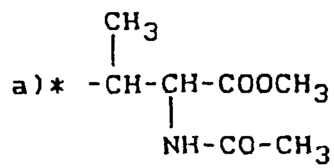 | Öl | *1) 0,17/0,22 *3) 0,27/0,32 |
| 28 | 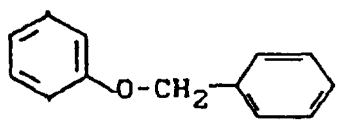 | $H_3C$-OOC | 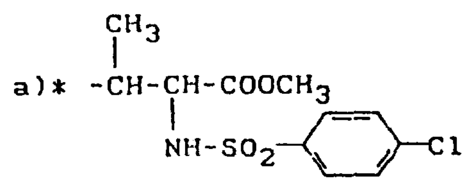 | Öl | *6) 0,42/0,46 |
| 29 | 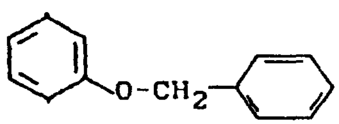 | $H_3C$-OOC | 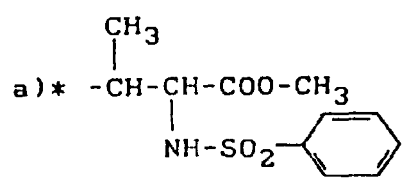 | Öl | *6) 0,32/0,36 |

(a = R, S; b = S, R)

| Beisp.-Nr. | $R^1$ | $R^2$ | Y | Fp.: [°C] | $R_f$ (Laufmittel) |
|---|---|---|---|---|---|
| 30 | 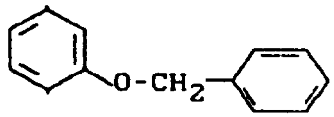 | 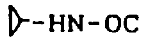 | a)* 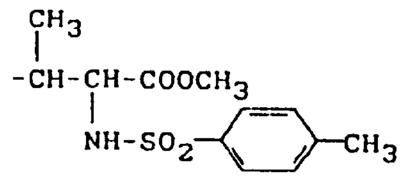 | Öl | *3) 0,46/0,32 |
| 31 | 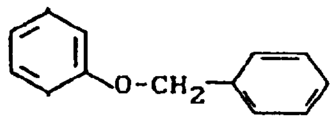 | 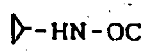 | a)* 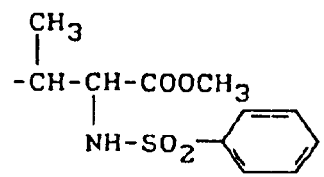 | Schaum | *7) 0,44/0,5 *3) 0,35/0,4 |
| 32 | 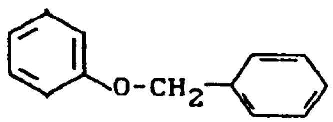 | 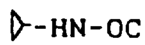 | a)* 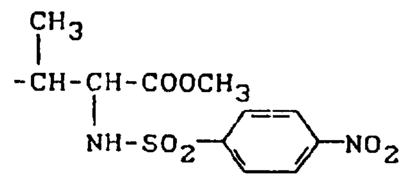 | Schaum | *7) 0,59/0,65 *3) 0,49/0,55 |
| 33 | 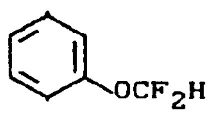 | $NO_2$ | a)* 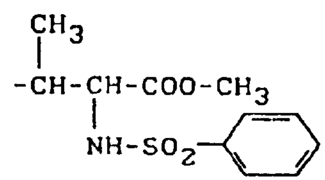 | 194-197 | *2) 0,45 |

(a = R, S; b = S, R)

| Beisp.-Nr. | $R^1$ | $R^2$ | Y | Fp.: [°C] | $R_f$ (Laufmittel) |
|---|---|---|---|---|---|
| 34 | 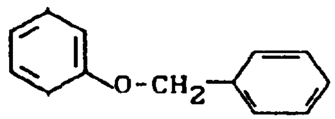 | 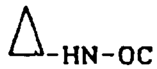 | a)* 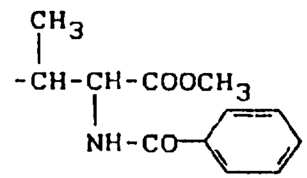 | Schaum | *2) 0,02<br>*3) 0,42 |
| 35 | 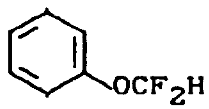 | $NO_2$ | a)* 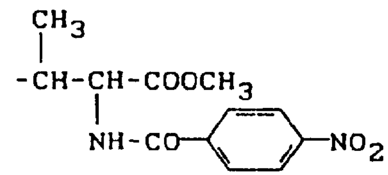 | Schaum | *1) 0.39 |
| 36 | 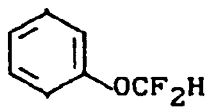 | $NO_2$ | a)* 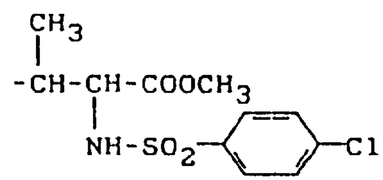 | 180 | *2) 0,55 |
| 37 | 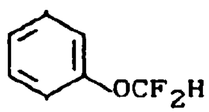 | $NO_2$ | a)* 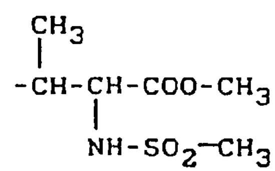 | Öl | *1) 0,26 |

(a = R, S; b = S, R)

| Beisp.-Nr. | $R^1$ | $R^2$ | Y | Fp.: [°C] | $R_f$ (Laufmittel) |
|---|---|---|---|---|---|
| 38 | 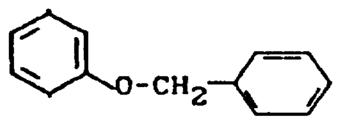 | $H_3C-OOC$ | a)* 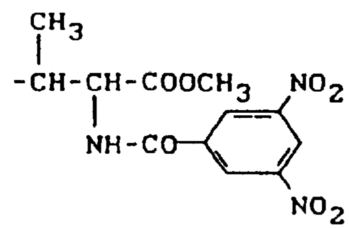 | 157-159 | *1) 0.29/0.33<br>*8) 0.39/0.47 |
| 39 | 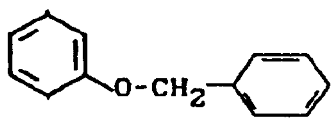 | $H_3C-OOC$ | a)* 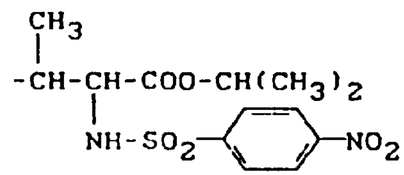 | Schaum | *4) 0,37/0,41 |
| 40 | 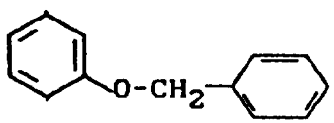 | $H_3C-OOC$ | a)* 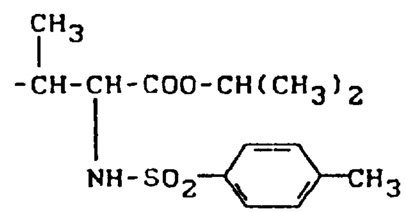 | Schaum | *1) 0,67<br>*9) 0,18 |
| 41 | 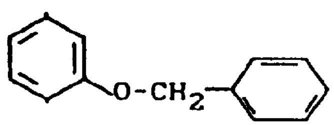 | $H_3C-OOC$ | a)* 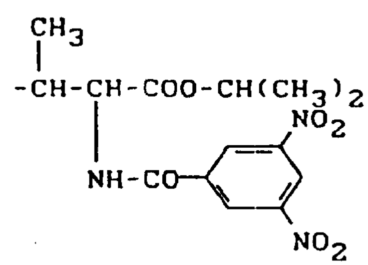 | Schaum | *2) 0,76/0,82 |

(a = R, S; b = S, R)

| Beisp.-Nr. | $R^1$ | $R^2$ | Y | Fp.: [°C] | $R_f$ (Laufmittel) |
|---|---|---|---|---|---|
| 42 | 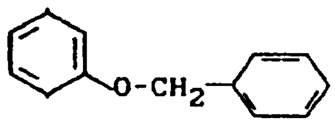 | 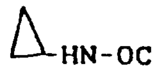 | a)* 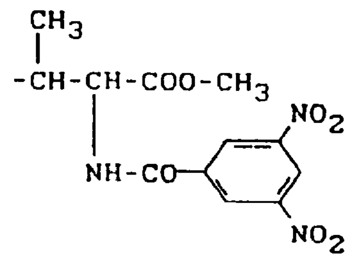 | 155-157 | |
| 43 | 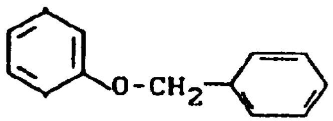 | $H_5C_2$-HN-OC | a)* 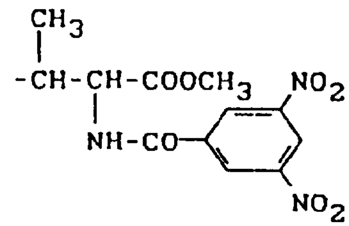 | 118-122 | *2) 0,05<br>*3) 0,71 |
| 44 | 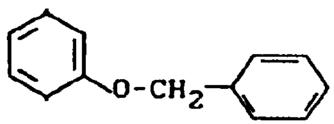 | $H_5C_2$-HN-OC | a)* 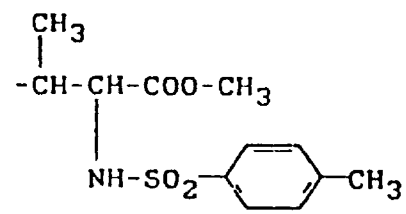 | | *7) 0,43/0,5<br>*3) 0,46/0,52 |
| 45 | 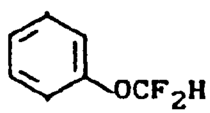 | $NO_2$ | b)* 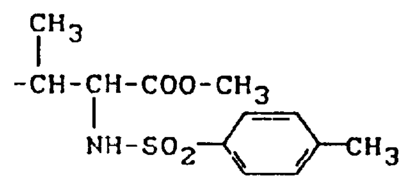 | 178 | *9) 0,48 |

(a = R, S; b = S, R)

| Beisp.-Nr. | $R^1$ | $R^2$ | Y | Fp.: [°C] | $R_f$ (Laufmittel) |
|---|---|---|---|---|---|
| 46 | 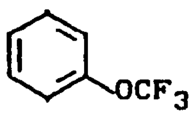 | $NO_2$ | b)* 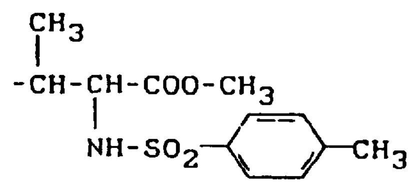 | 186-188 | *2) 0,56 |
| 47 | 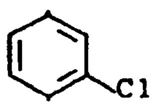 | 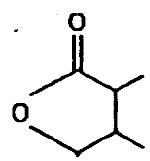 | a)* 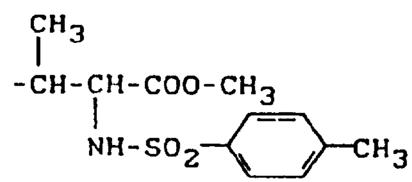 | >270 | |

(a = R, S; b = S, R)

## Zeichenerklärung der Tabellen

*1) Toluol/Essigester 1:1

*2) Methylenchlorid/Essigester 9:1

*3) Methylenchlorid/Essigester 1:4

*4) Methylenchlorid/Essigester 20:1

*5) Chloroform/Essigester 9:1

*6) Toluol/Essigester 2:1

*7) Essigester

*8) Toluol/Methylisobutylketon 3:1

*9) Toluol/Essigester 4:1

*10) Toluol/Essigester 1:5

## Patentansprüche

1. Dihydropyridinderivate der Formel I

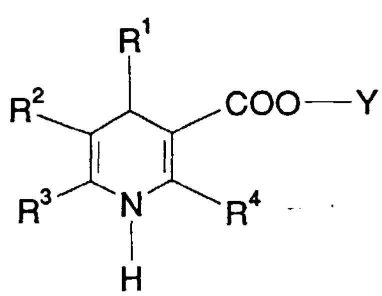

(I)

in welcher

$R^1$ - für geradkettiges oder verzweigtes Alkyl mit bis zu 4 Kohlenstoffatomen steht, oder
- für Cyclopropyl, Cyclopentyl oder Cyclohexyl steht, oder

- für Phenyl steht, das bis zu 2-fach gleich oder verschieden substituiert sein kann durch Nitro, Cyano, Trifluormethyl, Fluor, Chlor, Methyl, Methoxy, Trifluormethoxy, Difluormethoxy, gegebenenfalls durch Methyl, Fluor oder Chlor substituiertes Phenylsulfonyloxy, oder durch $C_1$-$C_4$-Alkoxy oder $C_1$-$C_4$-Alkylthio, die jeweils substituiert sein können durch Cyclohexyl oder Phenyl, das seinerseits durch Methyl, Fluor, Chlor oder Methoxy substituiert sein kann, oder
- für Pyridyl steht, und

$R^2$ - für Nitro, Cyano oder - für eine Gruppe der Formel COO-Z-$R^5$ oder

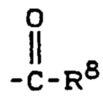

steht, worin

$R^5$ - für Wasserstoff oder $C_1$-$C_6$-Alkyl steht, das gegebenenfalls durch Sauerstoff oder Schwefel unterbrochen sein kann und das gegebenenfalls durch Hydroxy, $C_1$-$C_4$-Alkoxycarbonyl oder Carboxy, oder durch Fluor, Chlor, Methyl oder Methoxy substituiertes Phenyl oder Phenoxy substituiert sein kann, oder - für eine Gruppe der Formel

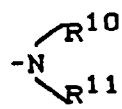

steht,
worin

$R^{10}$ und $R^{11}$ gleich oder verschieden sind und jeweils

- für Wasserstoff, für geradkettiges oder verzweigtes Alkyl mit bis zu 6 Kohlenstoffatomen stehen, das substituiert sein kann durch Fluor, Chlor, Hydroxy, Ethoxy, Methoxy, Carboxy, $C_1$-$C_4$-Alkoxycarbonyl oder durch Phenyl, welches seinerseits durch Trifluormethyl, Trifluormethoxy, $C_1$-$C_2$-Alkyl, Fluor, Chlor oder $C_1$-$C_2$-Alkoxy substituiert sein kann, oder
- für Cyclopropyl, Cyclopentyl oder Cyclohexyl stehen, oder
- für Phenyl stehen, das durch Fluor, Chlor, $C_1$-$C_2$-Alkyl, $C_1$-$C_2$-Alkoxy, Trifluormethyl, Trifluormethoxy, Difluormethoxy, Trifluormethylthio, Amino, $C_1$-$C_2$-Alkylamino substituiert sein kann, oder

$R^{10}$ und $R^{11}$ zusammen für Piperidinyl, Morpholinyl, Methyl-piperazinyl oder N-Benzylpiperazinyl stehen,

und

Z - für eine einfache Bindung oder eine geradkettige oder verzweigte Alkylenkette mit bis zu 6 C-Atomen steht
und

$R^8$
- für geradkettiges oder verzweigtes Alkyl mit bis zu 4 Kohlenstoffatomen steht, oder
- für die Gruppe

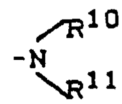

steht,
worin
$R^{10}$ und $R^{11}$ die oben angegebene Bedeutung haben,

und

$R^3$ - für geradkettiges, verzweigtes oder cyclisches Alkyl mit bis zu 4 Kohlenstoffatomen steht, oder

$R^2$ und $R^3$ gemeinsam einen Ring der Formel

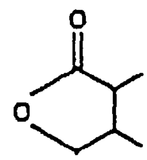

bilden,
und

$R^4$
- für geradkettiges oder verzweigtes Alkyl mit bis zu 4 Kohlenstoffatomen steht, oder
- für Cyclopropyl steht,
und

Y - für einen Rest

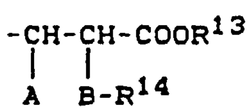

steht,
worin

A - für Wasserstoff oder Methyl steht,
und

B - für eine Gruppe der Formel -NH-, -NH-CO-, -NH-COO- oder $-NH-SO_2$ steht,
und

$R^{13}$ - für geradkettiges oder verzweigtes Alkyl mit bis zu 6 Kohlenstoffatomen steht, das substituiert sein kann durch Hydroxy, Methoxy oder Phenyl,
und

$R^{14}$ - Wasserstoff bedeutet, oder
- für geradkettiges oder verzweigtes Alkyl mit bis zu 6 Kohlenstoffatomen steht, das substituiert sein kann durch Hydroxy, $C_1$-$C_2$-Alkoxycarbonyl oder durch Phenyl, das seinerseits substituiert sein kann durch Methyl, Trifluormethyl, Fluor oder Chlor, oder
- für Cyclopropyl, Cyclopentyl oder Cyclohexyl steht, oder

- für Phenyl steht, das bis zu 2-fach gleich oder verschieden substituiert sein kann durch Nitro, Cyano, Fluor, Chlor, $C_1$-$C_2$-Alkyl, $C_1$-$C_4$-Alkoxy, $C_1$-$C_2$-Alkylthio, Carbamoyl, Carboxy, $C_1$-$C_4$-Alkoxycarbonyl, Trifluormethyl, Trifluormethoxy, $C_1$-$C_2$-Alkylsulfamoyl oder $C_1$-$C_2$-Alkylamino, oder
- für Pyridyl steht,

sowie deren Salze.

**2.** Verfahren zur Herstellung von Verbindungen der allgemeinen Formel I gemäß Anspruch 1, dadurch gekennzeichnet, daß man

[A] Aldehyde der allgemeinen Formel III

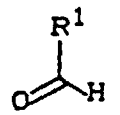 (III)

in welcher
$R^1$ die oben angegebene Bedeutung hat,
und Verbindungen der Formel (II)

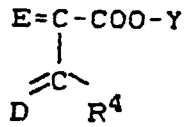 (II)

in welcher

R⁴ und Y die oben angegebene Bedeutung haben,

D - für Sauerstoff steht
und

E in diesem Fall für zwei Wasserstoffatome steht,

gegebenenfalls nach Isolierung der entstehenden Benzylidenderivate mit Aminocrotonsäurederivaten der allgemeinen Formel (IV)

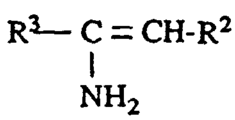 (IV)

in welcher
$R^2$ und $R^3$ die oben angegebene Bedeutung haben,
umsetzt,
oder indem man

[B] Aldehyde der allgemeinen Formel (III)

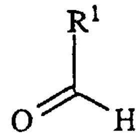

(III)

in welcher
$R^1$ die oben angegebene Bedeutung hat,
mit Verbindungen der allgemeinen Formel (V)

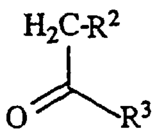

(V)

in welcher
$R^2$ und $R^3$ die oben angegebene Bedeutung haben,
entweder direkt oder nach Isolierung der entstehenden Benzylidenderivate der allgemeinen Formel (VI)

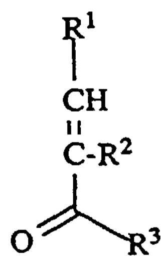

(VI)

in welcher
$R^1$, $R^2$ und $R^3$ die oben angegebene Bedeutung haben,
mit Verbindungen der allgemeinen tautomeren Formeln (II)

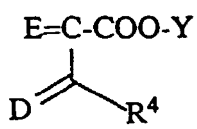

(IIa)

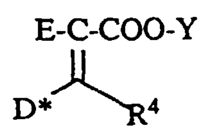

(IIb)

in welcher

| | |
|---|---|
| $R^4$ und Y | die oben angegebene Bedeutung haben, |
| D | für NH steht, |
| D* | für NH steht, und |
| E | für Wasserstoff steht, umsetzt. |

**3.** Verwendung der Verbindungen der allgemeinen Formel I gemäß Anspruch 1 zur Herstellung von Dihydropyridincarbonsäuren in Racematform oder als reine Enantiomere der allgemeinen Formel XI

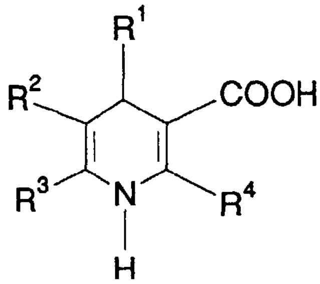

(XI)

in welcher
$R^1$, $R^2$, $R^3$ und $R^4$ die in Anspruch 1 angegebene Bedeutung haben,
dadurch gekennzeichnet, daß man den Rest Y nach üblichen Methoden aus Verbindungen der allgemeinen Formel I abspaltet.

**Claims**

**1.** Dihydropyridine derivatives of the formula I

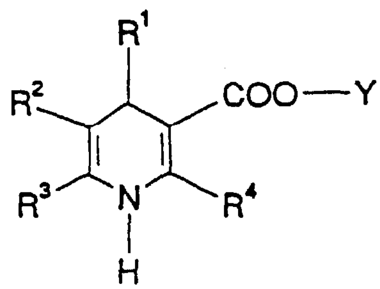

(I)

in which

- $R^1$ - represents straight-chain or branched alkyl having up to 4 carbon atoms, or - represents cyclopropyl, cyclopentyl or cyclohexyl, or
  - represents phenyl which can be monosubstituted or disubstituted by identical or different nitro, cyano, trifluoromethyl, fluorine, chlorine, methyl, methoxy, trifluoromethoxy, difluoromethoxy or phenylsulphonyloxy which is optionally substituted by methyl, fluorine or chlorine, or by $C_1$-$C_4$-alkoxy or $C_1$-$C_4$-alkylthio which can each be substituted by cyclohexyl or phenyl which, in turn, can be substituted by methyl, fluorine, chlorine or methoxy, or
  - represents pyridyl, and

- $R^2$ - represents nitro, cyano or
  - a group of the formula
    COO-Z-$R^5$ or

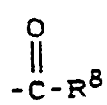

,

wherein

$R^5$ - represents hydrogen or $C_1$-$C_6$-alkyl which can optionally be interrupted by oxygen or sulphur and which can optionally be substituted by hydroxyl, $C_1$-$C_4$-alkoxycarbonyl or carboxyl, or by

phenyl or phenoxy, each of which is substituted by fluorine, chlorine, methyl or methoxy, or

- represents a group of the formula

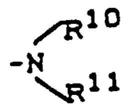

wherein

$R^{10}$ and $R^{11}$ are identical or different and each

- represent hydrogen, straight-chain or branched alkyl having up to 6 carbon atoms and which can be substituted by fluorine, chlorine, hydroxyl, ethoxy, methoxy, carboxyl or $C_1$-$C_4$-alkoxycarbonyl, or by phenyl which, in turn, can be substituted by trifluoromethyl, trifluoromethoxy, $C_1$-$C_2$-alkyl, fluorine, chlorine or $C_1$-$C_2$-alkoxy, or
- represent cyclopropyl, cyclopentyl or cyclohexyl, or
- represent phenyl which can be substituted by fluorine; chlorine, $C_1$-$C_2$-alkyl, $C_1$-$C_2$-alkoxy, trifluoromethyl, trifluoromethoxy, difluoromethoxy, trifluoromethylthio, amino or $C_1$-$C_2$-alkylamino, or

$R^{10}$ and $R^{11}$ together represent piperidinyl, morpholinyl, methylpiperazinyl or N-benzylpiperazinyl,

and

Z - represents a single bond or a straight-chain or branched alkylene chain having up to 6 C atoms
and

$R^8$
- represents straight-chain or branched alkyl having up to 4 carbon atoms, or
- represents the group

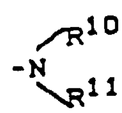

wherein
$R^{10}$ and $R^{11}$ have the abovementioned meaning,

and

$R^3$ - represents straight-chain, branched or cyclic alkyl having up to 4 carbon atoms,
or

$R^2$ and $R^3$ jointly form a ring of the formula

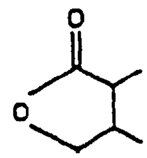

and

R4 - represents straight-chain or branched alkyl having up to 4 carbon atoms, or
- represents cyclopropyl,

and

Y - represents a radical

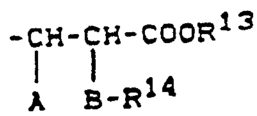

wherein

A - represents hydrogen or methyl,
and

B - represents a group of the formula -NH-, -NH-CO-, -NH-COO- or -NH-$SO_2$-,
and

$R^{13}$ - represents straight-chain or branched alkyl having up to 6 carbon atoms which can be substituted by hydroxyl, methoxy or phenyl,
and

$R^{14}$
- denotes hydrogen, or
- represents straight-chain or branched alkyl having up to 6 carbon atoms and which can be substituted by hydroxyl or $C_1$-$C_2$-alkoxycarbonyl, or by phenyl which, in turn, can be substituted by methyl, trifluoromethyl, fluorine or chlorine, or
- represents cyclopropyl, cyclopentyl or cyclohexyl, or
- represents phenyl which can be monosubstituted or disubstituted by identical or different nitro, cyano, fluorine, chlorine, $C_1$-$C_2$-alkyl, $C_1$-$C_4$-alkoxy, $C_1$-$C_2$-alkylthio, carbamoyl, carboxyl, $C_1$-$C_4$-alkoxycarbonyl, trifluoromethyl, trifluoromethoxy, $C_1$-$C_2$-alkylsulphamoyl or $C_1$-$C_2$-alkylamino, or - represents pyridyl,

and their salts.

**2.** Process for the preparation of compounds of the general formula I according to Claim 1, characterized in that

[A] aldehydes of the general formula (III)

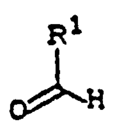 (III)

in which

$R^1$ has the abovementioned meaning,
and compounds of the formula (II)

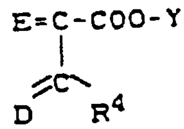

(II)

in which

$R^4$ and Y have the abovementioned meaning,

D - represents oxygen
and

E in this case represents two hydrogen atoms,
are reacted, optionally after isolation for the resulting benzylidene derivatives, with aminocrotonic acid derivatives of the general formula (IV)

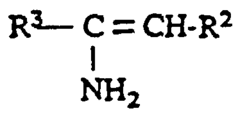

(IV)

in which
$R^2$ and $R^3$ have the abovementioned meaning, or

[B] aldehydes of the general formula (III)

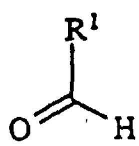

(III)

in which
$R^1$ has the abovementioned meaning,
and compounds of the general formula (V)

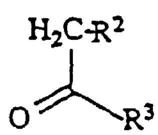

(V)

in which
$R^2$ and $R^3$ have the abovementioned meaning,
are reacted, either directly or after isolation of the resulting benzylidene derivatives of the general formula (VI)

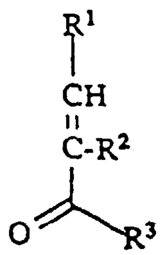 (VI)

in which
$R^1$, $R^2$ and $R^3$ have the abovementioned meaning,
with compounds of the general tautomeric formulae (II)

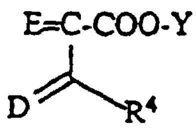
(IIa)

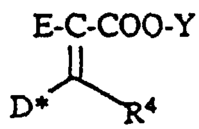
(IIb)

in which

| | |
|---|---|
| $R^4$ and Y | have the abovementioned meaning, |
| D | represents NH, |
| D* | represents $NH_2$, and |
| E r | epresents hydrogen. |

**3.** Use of the compounds of the general formula I according to Claim 1 for the preparation of dihydropyridinecarboxylic acids in racemate form or as pure enantiomers of the general formula XI

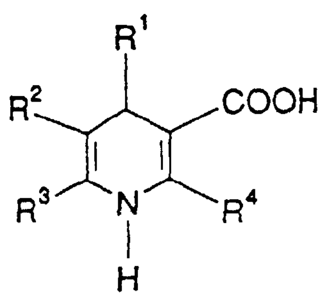 (XI)

in which
$R^1$, $R^2$, $R^3$ and $R^4$ have the meaning given in Claim 1,
characterized in that the radical Y is removed by customary methods from compounds of the general formula I.

**Revendications**

**1.** Dérivés de dihydropyridine de formule I

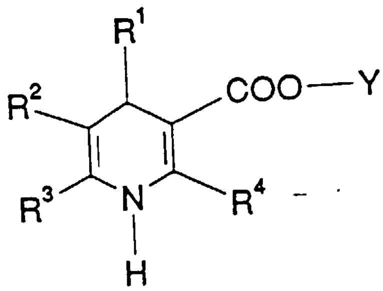

(I)

dans laquelle

$R^1$ - représente un groupe alkyle linéaire ou ramifié ayant jusqu'à 4 atomes de carbone, ou
- représente un groupe cyclopropyle, cyclopentyle ou cyclohexyle, ou
- représente un groupe phényle qui peut être substitué jusqu'à 2 fois identiques ou différentes par un reste nitro, cyano, trifluorométhyle, fluoro, chloro, méthyle, méthoxy, trifluorométhoxy, difluorométhoxy, par un reste phénylsulfonyloxy éventuellement substitué par un radical méthyle, fluoro ou chloro, ou par un reste alkoxy en $C_1$ à $C_4$ ou un reste alkylthio en $C_1$ à $C_4$ dont chacun peut être substitué par un radical cyclohexyle ou phényle qui peut lui-même être substitué par un radical méthyle, fluoro, chloro ou méthoxy, ou
- un groupe pyridyle, et

$R^2$ - représente un groupe nitro, cyano ou
- un groupe de formule
$COO\text{-}Z\text{-}R^5$ ou

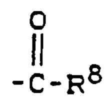

dans laquelle

$R^5$ - représente de l'hydrogène ou un groupe alkyle en $C_1$ à $C_6$ qui peut éventuellement être interrompu par de l'oxygène ou du soufre et qui peut être substitué, le cas échéant, par un reste hydroxy, (alkoxy en $C_1$ à $C_4$)carbonyle ou carboxy, ou un reste phényle ou phénoxy substitué par un radical fluoro, chloro, méthyle ou méthoxy, ou bien
- représente un groupe de formule

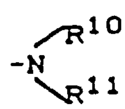

dans laquelle

$R^{10}$ et $R^{11}$ sont identiques ou différents et représentent chacun

- de l'hydrogène, un groupe alkyle linéaire ou ramifié ayant jusqu'à 6 atomes de carbone, qui peut être substitué par un radical fluoro, chloro, hydroxy, éthoxy, méthoxy, carboxy, (alkoxy en $C_1$ à $C_4$)-carbonyle ou par un reste phényle qui peut lui-même être substitué par un radical trifluorométhyle, trifluorométhoxy, alkyle en $C_1$ ou $C_2$, fluoro, chloro ou alkoxy en $C_1$ ou $C_2$, ou bien
- représentent chacun un groupe cyclopropyle, cyclopentyle ou cyclohexyle,

ou bien

- représentent chacun un groupe phényle qui peut être substitué par un radical fluoro, chloro, alkyle en $C_1$ ou $C_2$, alkoxy en $C_1$ ou $C_2$, trifluorométhyle, trifluorométhoxy, difluorométhoxy, trifluorométhylthio, amino, alkylamino en $C_1$ ou $C_2$, ou bien

$R^{10}$ et $R^{11}$ forment ensemble un noyau pipéridinyle, morpholinyle, méthylpipérazinyle ou N-benzylpipérazinyle,

et

Z - représente une liaison simple ou une chaîne alkylénique linéaire ou ramifiée ayant jusqu'à 6 atomes de carbone

et

$R^8$
- représente un groupe alkyle linéaire ou ramifié ayant jusqu'à 4 atomes de carbone, ou
- représente le groupe

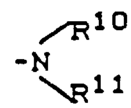

dans lequel

$R^{10}$ et $R^{11}$ ont la définition indiquée ci-dessus,

et

$R^3$ - représente un groupe alkyle linéaire, ramifié ou cyclique ayant jusqu'à 4 atomes de carbone, ou bien

$R^2$ et $R^3$ forment ensemble un noyau de formule

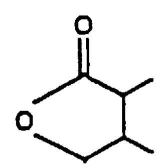

et

$R^4$
- représente un groupe alkyle linéaire ou ramifié ayant jusqu'à 4 atomes de carbone, ou
- représente un groupe cyclopropyle,

et

Y - représente un reste de formule

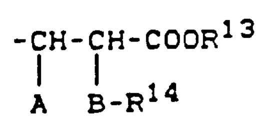

dans laquelle

A - représente de l'hydrogène ou un groupe méthyle,

et

B - représente un groupe de formule -NH-, -NH-CO-, -NH-COO- ou $-NH-SO_2-$,

et

$R^{13}$ - représente un groupe alkyle linéaire ou ramifié ayant jusqu'à 6 atomes de carbone, qui peut

être substitué par un radical hydroxy, méthoxy ou phényle,
et

$R^{14}$
- désigne de l'hydrogène, ou
- représente un groupe alkyle linéaire ou ramifié ayant jusqu'à 6 atomes de carbone, qui peut être substitué par un reste hydroxy, (alkoxy en $C_1$ ou $C_2$)-carbonyle ou par un reste phényle qui peut lui-même être substitué par un radical méthyle, trifluorométhyle, fluoro ou chloro, ou
- représente un groupe cyclopropyle, cyclopentyle ou cyclohexyle, ou
- représente un groupe phényle qui peut être substitué jusqu'à 2 fois identiques ou différentes par un radical nitro, cyano, fluoro, chloro, alkyle en $C_1$ ou $C_2$, alkoxy en $C_1$ à $C_4$, alkylthio en $C_1$ ou $C_2$, carbamoyle, carboxy, (alkoxy en $C_1$ à $C_4$)-carbonyle, trifluorométhyle, trifluorométhoxy, alkylsulfamoyle en $C_1$ ou $C_2$ ou alkylamino en $C_1$ ou $C_2$, ou
- représente un groupe pyridyle,

ainsi que leurs sels.

**2.** Procédé de production de composés de formule générale I suivant la revendication 1, caractérisé en ce que :

[A] on fait réagir des aldéhydes de formule générale III

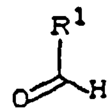

(III)

dans laquelle
$R^1$ a la définition indiquée ci-dessus, et des composés de formlule (II)

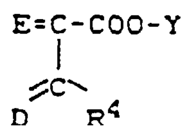

(II)

dans laquelle

| | |
|---|---|
| $R^4$ et Y | ont la définition indiquée ci-dessus, |
| D | - représente de l'oxygène<br>et |
| E | représente dans ce cas deux atomes d'hydrogène, éventuellement après isolement des dérivés benzylidéniques produits, avec des dérivés d'acide aminocrotonique de formule générale (IV) |

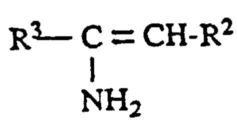

(IV)

dans laquelle
$R^2$ et $R^3$ ont la définition indiquée ci-dessus, ou par le fait que

[B] on fait réagir des aldéhydes de formule générale (III)

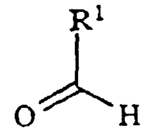

(III)

dans laquelle
$R^1$ a la définition indiquée ci-dessus, avec des composés de formule générale (V)

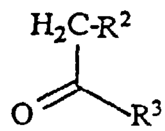

(V)

dans laquelle
$R^2$ et $R^3$ ont la définition indiquée ci-dessus, directement ou après isolement des dérivés benzylidéniques produits de formule générale (VI)

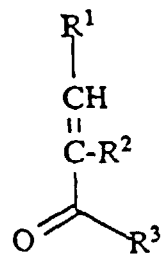

(VI)

dans laquelle
$R^1$, $R^2$ et $R^3$ ont la définition indiquée ci-dessus, avec des composés de formules générales tautomères (II)

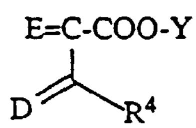

(IIa)

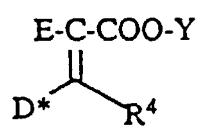

(IIb)

dans lesquelles

| | |
|---|---|
| $R^4$ et Y | ont la définition indiquée ci-dessus, |
| D | est un groupe NH, |
| D* | est un groupe NH, et |
| E | représente de l'hydrogène. |

**3.** Utilisation des composés de formule générale I suivant la revendication 1 pour la production d'acides dihydropyridinecarboxyliques sous la forme de racémate ou comme énantiomères purs de formule générale XI

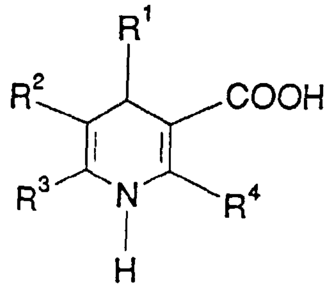

(XI)

dans laquelle

$R^1$, $R^2$, $R^3$ et $R^4$ ont la définition indiquée dans la revendication 1,

caractérisé en ce qu'on élimine par des moyens classiques le reste Y des composés de formule générale I.